Europäisches Patentamt

European Patent Office

Office européen des brevets

(11) Publication number: **0 011 924**
**A1**

## EUROPEAN PATENT APPLICATION

(21) Application number: **79302312.8**

(22) Date of filing: **23.10.79**

(51) Int. Cl.³: **A 61 L 9/12**
**B 65 D 81/24**

(30) Priority: **23.10.78 GB 4157378**

(43) Date of publication of application:
**11.06.80 Bulletin 80/12**

(84) Designated Contracting States:
**AT BE CH DE FR GB IT LU NL SE**

(71) Applicant: **BUSH BOAKE ALLEN Limited**
**Blackhorse Lane Walthamstow**
**London E17 5QP(GB)**

(72) Inventor: **Ansari, Hifzur Rahman**
**21, Derwent Avenue Rayleigh**
**Essex(GB)**

(72) Inventor: **Williams, Philip Leonard**
**8 Lakeside Crescent**
**Barnet Hertfordshire(GB)**

(74) Representative: **Savidge, Roger Gordon**
**Madgwick et al,**
**c/o Albright & Wilson Limited 1 Knightsbridge Green**
**London SW1X 7QD(GB)**

(54) Polymeric fragrance materials and processes for their manufacture.

(57) The invention provides a sealed product comprising synthetic hydrophilic polymer compositions containing a volatile active material which is released on exposure to the atmosphere which are wrapped in a transparent film which is impermeable to water vapour. Examples of packaging films which are useful are:

Barex/polythene and barex/polypropylene duplex laminates, PVC/PVDC duplex laminates PV/PVDC/polyethylene and PVC/PVDC/polypropylene triplex laminates and polyester/PVC/polyethylene triplex laminates.

These products exhibit a lesser tendency to exude the volatile material even on prolonged storage as compared to previously known products which were wrapped in polyethylene films. In a preferred form these products are produced by preparing a blister pack from any suitable material and using this as a mould into which the monomers are poured and then polymerised. Such products are readily adapted so as to be sold without further packaging or decoration.

./...

Croydon Printing Company Ltd.

FIG.1

This invention relates to synthetic hydrophilic polymer compositions which comprise an entrapped volatile active ingredient which is gradually released when the composition is exposed to the atmosphere. The volatile material is mostly commonly a fragrance i.e. a natural essential oil or synthetic perfume or a blend thereof but volatile insecticides bacteriocides pheronomes and fabric softeners may be entrapped to provide products with a consequent variety of applications. All these products are customarily sealed within a package which is substantially impermeable to the volatile material in order to prevent its premature release.

A particular problem with systems based upon synthetic hydrophilic polymerised materials is the tendency of the volatile ingredient to exude from the polymer composition upon prolonged storage or exposure to high temperature producing a product which has an unattractive greasy or wet surface. The problem has been especially prevalent in products which were wrapped in a transparent polymeric film in order to maximise their visual appeal to the customer. We have now discovered that this problem of exudation may be alleviated using transparent packaging material which is sufficiently impermeable to water vapour as well as to the volatile substance. Thus the products according to our invention retain their visual appeal and remain functional even on prolonged storage. From one aspect our invention provides a synthetic hydrophilic polymeric composition containing a volatile active ingredient which is capable of controlled release to the atmosphere and a package enclosing said polymeric composition to an extent sufficient substantially to prevent loss of active ingredient said package comprising a transparent film which is substantially impermeable to the active ingredient, and which over at least part of the surface of the package, constitutes the sole barrier between the polymeric composition and the outside atmosphere, said film having a water permeability less than 5 gm/m$^2$ per day at 38$^o$C and 90% RH and sufficiently low substantially to prevent exudation of active ingredient on storage.

It will be appreciated that no packaging material suitable for this type of product is totally impermeable to the passage of water vapour. Hitherto transparent packaging films such as PVC film of 200 micron gauge having a permeability of from 5 to 6.0 gm/m$^2$ per day at 38$^o$ and 90% relative humidity (RH) has been commonly used and products enclosed in such a film suffer from a tendency to exude the volatile substance. In order to effectively suppress the exudation of a typical product which will be stored for some months prior to sale, we have now discovered that the product should be packaged in a film having a water transmission of less than 5 preferably less than 3 say 2 and most preferably less than 1 expressed in gm/m$^2$ per day at 38$^o$C and 90% RH. The maximum water permeability which can be tolerated depends on the particular polymer composition and active material. Products having a high tendency to exude require a more water impermeable film in order to prevent high perceptible exudation.

The film must retain the properties of being transparent and being impermeable to the volatile substance in order to prevent the escape thereof. Where such a combination of properties is not possessed by a single film a laminate of two or more films may be used. The formulation of such laminates to produce a film having particular properties is a well known technique in the packaging art and certain laminates which are useful according to our invention are commercially available. Examples of packaging films presently available which are useful include:-

Barex*/polythene and Barex/polypropylene duplex laminates
(*Barex is a modified polyacrylonitrile film sold by the
Vistron Corporation); polyvinylchloride (PVC) polyvinylidene
chloride (PVDC) duplex laminates; PVC/PVDC/polyethylene
and PVC/PVDC/polypropylene triplex laminates and polyester/
PVDC/PE triplex laminates

It will be appreciated that the packaging film selected for a particular product may vary according to the tendency of the volatile material to exude from the particular polymeric material and the conditions to which the package is to be subjected prior to its sale.

In particular products which are to be sold in countries where hot dry weather prevails exhibit a greater tendency to exude the volatile material than when they are sold in countries having a temperate climate and thus will normally be packaged in a material having a lower permeability to water vapour. Clearly, whatever the conditions under which the product is to be manufactured stored and sold the packaging film must retain its impermeability to the volatile material.

The synthetic polymer compositions to which this invention relates are polymers of alpha-beta or beta-gamma unsaturated carboxylic esters which possesses hydrophilic functions such as, for example, alkoxy or, preferably, hydroxy groups. Typical monomers are hydroxyethyl acrylate, or preferably hydroxyethyl methacrylate, and the hydroxypropyl, hydroxybutyl and glyceryl esters of acrylic or methacrylic acid. Polyoxyalkylated derivatives of the foregoing monomers may also be used, such as polyoxyethylene acrylate or polyoxypropylene glyceryl monomethacrylate. Usually the number of oxyalkylene groups is up to 20 although higher alkoxylates are operative and may be preferred for certain specialised applications. Glycidyl esters of unsaturated fatty acids may also be used as monomers.

The monomer mixture preferably contains a minor proportion of one or more bi- or poly-functional monomers, i.e. monomers having two or more polymerizable unsaturated groups in the molecule. These act as cross-linking agents and have the effect of increasing the hardness and rigidity of the polymeric product. Examples are the acrylates and methacrylates of polyhydroxylic compounds such as ethylene glycol, propylene glycol, 1,3-butylene glycol, 1,6-hexanediol, glycerol, pentaerythritol and trimethylolpropane, in each case at least two of the hydroxyl groups being esterified with acrylic or methacrylic acid. A particularly preferred cross-linking agent is trimethylolpropane trimethacrylate. Alkoxylated derivatives of hydroxy-containing polyfunctional monomers, and glycidyl esters are also operative.

The prepolymer may optionally contain some non-hydrolic monomers such as ethyl methacrylate or isobutyl acrylate. It may also comprise small proportions of other vinylic monomers such vinyl chloride and styrene.

If required, a firmer texture may be produced by adjustment of the proportion of cross-linking agent in the monomer mixture. Additionally, the hardness and flexibility of the product depend on the amount and nature of the active ingredient and the solubilising

agent present, and the proportion of cross-linking agent may be varied
to compensate for these effects also. No cross-linking agent need be
added if a very soft polymer is required. However, some cross-linking
agent will usually be included, typically up to 25% e.g. 10 to 20%
by weight based on the total weight of monomers.

The polymer systems useful in the products of the present
invention preferably comprise a solubilising agent as is described
in our co-pending British Application 4896/77

The solubiliser is an organic compound which has both hydrophilic and lipophilic characteristics. It must be compatible with
the polymer, e.g. soluble therein to an extent of at least 5% based
on the weight of the polymer and preferably more compatible with the
volatile material than is the polymer. Preferably the solubiliser
has molecular weight less than 1200 most preferably less than 1000.

The solubilising agent may be a surfactant, preferably a nonionic surfactant, such as, for example, a polyalkylene oxide, e.g.
polyethylene oxide or polypropylene oxide having from 2 to 25
oxyalkylene units or a polyoxyalkylene condensate with a fatty alcohol
or alkyl phenol or with a glycerol or sorbitol ester, or a polyoxyalkylene ester of a fatty acid. Typical examples include polyoxyethylene stearate, polyoxypropylene sorbitan monolaurate, polyoxyethylene glyceryl mono oleate and polyoxyethylene nonylphenol.
Glyceryl and sorbitan fatty esters themselves are similarly useful
as are the other commonly used nonionic surfactants including alkylol-
amides. It is sometimes convenient to employ lower molecular weight,
nonionic hydrotropes such as hydroxyethyl acetate, methyl hydroxyethyl
ketone, methyl hydroxyethyl ether or propanediol. Preferably the
solubilising agent has at least one hydroxyl group and at least one
alkyl group which preferably has from 3 to 18, most preferably 3
to 10 carbon atoms. The solubilising agent may additionally comprise for example, ether, keto, ester, amide, amine, aryl and/or
cyclo aliphatic groups. We prefer for most purposes that the solubilising agent should be sufficiently involatile not to evaporate to any appreciable extent from the polymer. However, for certain specialised purposes we have discovered that there may be an advantage in using solubilising agents which comprise volatile substances. It is often convenient to use mixtures of solubilising agents, especially mixtures of solubilising agents, having significantly different HLB values.

Selection of the optimum solubilising agent depends on the nature of the active ingredient as well as the hydrophilic or other character of the resin. For example the solubilising agent may be chosen according to its HLB value so as to be compatible with the volatile materials and with the polymer. Thus for highly lipophilic volatile materials it is desirable to employ the more lipophilic solubilising agents, and preferably to make the polymer more lipophilic in character by lowering the proportion of hydroxyl groups, or to use a mixture of a relatively lipophilic and a relatively hydrophilic solubiliser.

Although nonionic solubilisers are strongly preferred it is sometimes possible or even advantageous to use cationic surfactants or hydrotropes as at least part of the solubiliser, such as quaternary ammonium salts or imidazolines.

The proportion of solubiliser is chosen, preferably with the range 4 to 200 per cent based on the amount of monomer used to prepare the polymer according to the requirements of the system including the relative compatibility of the volatile material and the polymer, the amount of volatile material to be incorporated and the desired rigidity of the polymer and rate of release of the volatile material. Large amounts of solubiliser tend to soften the polymer, increase the capacity for volatile material and its rate of release and the ease of forming a clear resin.

The invention is particularly applicable to volatile materials which are fragrances, including natural essential oils and synthetic perfumes, and blends thereof.

BAD ORIGINAL

Typical perfumery materials which may form part of, or possibly
the whole of, the active ingredient include: natural essential oils
such as lemon oil, mandarin oil, clove leaf oil, petitgrain oil, cedar
wood oil, patchouli oil, lavandin oil, neroli oil, ylang oil, rose
absolute or jasmin absolute; natural resins such as labdanum resin
or olibanum resin; single perfumery chemicals which may be isolated
from natural sources or manufactured synthetically, as for example
alcohols such as geraniol, nerol, citronellol, linalol, tetrahydro-
geraniol, betaphenylethyl alcohol, methyl phenyl carbinol, dimethyl
benzyl carbinol, menthol or cedrol; acetates and other esters derived
from such alcohols; aldehydes such as citral, citronellal, hydroxy-
citronellal, lauric aldehyde, undecylenic aldehyde, cinnamaldehyde,
amyl cinnamic aldehyde, vanillin or heliotropin; acetals derived
form such aldehydes; ketones such as methyl hexyl ketone, the ionones
and the methylionones; phenolic compounds such as eugenol and isoeu-
genol; synthetic musks such as musk xylene, musk ketone and ethylene
brassylate; and other materials commonly employed in the art of
perfumery. Typically at least five, and usually at least ten, of such
materials will be present as components of the active ingredient.

The volatile material is most commonly a fragrance material
but volatile insecticides, bacteriocides, pheromones and fabric
softeners can also usefully be incorporated.

The proportion of volatile material which is incorporated in
the polymer may range from the minimum effective concentration, which
will depend on the particular volatile material, up to the maximum
amount that can be incorporated in the resin, depending on the compatibility of the volatile material with the polymer and the nature
and amount of the solubilising agent. It has been found possible,
to incorporate up to 200% of some volatile material, based on the
weight of the polymer. Other volatile materials may provide
useful results in proportions as small as 100 ppm or even less.
Typically, however, the proportion of volatile material is between 2.5% and 100% based on the weight of the polymer, e.g. 5 to 50%.

The compositions of our invention usually contain water. The presence of some water is generally desirable as a vehicle for the catalyst system used in preparing the polymer. The presence of water also tends to accelerate the volatilisation of the volatile materials and is, therefore, often desirable in amounts up to the maximum capacity of the system, which in practice may be up to 50% by weight of the polymer. Typically the amount of water is between 1 and 40% by weight of the polymer. More usually the amount of water is from 2 to 10% by weight of the polymer.

The compositions of our invention may additionally contain relatively non-volatile additives such as colourings. Usually the compositions of our invention contain residues of the polymerisation catalyst or its degradation products.

Our novel polymers are conveniently prepared by mixing together the monomers and preferably solubilising agent and a polymerisation catalyst in the presence of the active ingredients and water. It is sometimes possible to polymerise the mixture without a catalyst, for example by heating or allowing the mixture to stand for a sufficient time, but for most practical purposes it is necessary to add a catalyst. In general the polymerisation is initiated by a free radical source, for example, an organic peroxide such as benzoyl peroxide, lauryl, perdicarbonate or isobutyl peroctoate, or an azo compound such as azoisobutyronitrile, which generate free radicals on heating. However, a problem which may arise with such initiators in systems with include volatile ingredients is that the elevated temperature required to initiate polmerisation in a commercially acceptable time, together with the heat released during the exothermic polymerisation reaction, can result in premature volatilisation of active ingredients or degradation of heat-sentive material We have devised catalyst systems which operate at lower temperatures and which, in some preferred cases, work

BAD ORIGINAL

satisfactorily at room temperature or below. These initiator systems belong to the general class of redox catalyst, which are combinations of oxidising and reducing agents.

The oxidising agent may be organic, such as one of the organic peroxides already mentioned, or inorganic, such as potassium or ammonium persulphate. A particularly preferred inorganic oxidixing agent is hydrogen peroxide, which has the residue of insoluble inorganic products in the polymer.

The oxidising agent may be used in conjunction with an inorganic reducing agent such as sodium thiosulphate or preferably an organic reducing agent such as ascorbic acid or araboascorbic acid (erythorbic acid). These last two are especially effective and may be used as polymerisation catalysts in conjunction with an oxidising agent, especially a persulphate, or most preferably, hydrogen peroxide.

Mixtures of ascorbic acid and hydrogen peroxide are capable of initiating polymerisation rapidly at room remperature, so avoiding excessive heating of the polymer. Cocatalysts including amines and salts of transition metals such as copper, cobalt, iron, nickel, and manganese are also useful.

The proportion of polymerisation catalyst is non critical, although higher proportions will produce shorter average chain lengths. For practical purposes it is not usually convenient to add more than about 5% by weight of catalyst based on the total mixture, preferably 0.1 to 2.5%.

Polymerisation is preferably effected under an inert atmosphere of, for example $CO_2$ or nitrogen, or under air tight sealing films to avoid inhibition of polymerisation at surface by oxygen. The polymerising mixture is preferably neutral or slightly acidic to water, and may benefit from the addition of small amounts of strong mineral acid especially when ascorbic acid and hydrogen peroxide is used
as the catalyst system. For example, a few drops of any strong
mineral acid which does not adversely affect the active ingredient
or polymer may be added to the polymerising mixture, usually the
most convenient being hydrochloric acid.

Polymers of this type may be manufactured in any shape or
form by pouring a liquid prepolymer into a suitable mould and
subsequently polymerising and/or curing the prepolymer to produce
a solid product. We have now discovered that a polymerised product
containing an entrapped volatile ingredient of the type to which
this invention relates can be simultaneously manufactured and
packaged for sale by shaping a transparent air impermeable film,
so as to form the mould. Thus from another aspect our invention
provides a process for the manufacture of a solid synthetic hydrophilic polymeric composition containing a volatile active ingredient
which process comprises forming a hollow mould from a substantially
air impermeable polymeric film having an opening at one end and introducing a liquid mixture which is capable on polymerisation and/or curing
of forming a solid polymeric product into the mould  and polymerising
and/or curing said mixture; the opening to the mould being  sealed
before during or after the polymerisation step.

Films formed from synthetic polymers such as polyvinyl chloride,
polyesters, polyacetals polyvinylidine chloride and polycarbonates are
suitable for forming the mould provided a film of suitable gauge e.g.
50 to 500 microns more usually 100 to 300 microns is employed.

The film used will preferably be substantially impermeable to the passage
of water vapour as hereinbefore defined. The mould is preferably formed
by shaping two pieces of a suitable film to form  opposite halves of
the mould. The two halves of the mould may be jointed by any technique

0011924

which produces a liquid tight joint.  Adhesives of various kinds may
be employed or heat may be applied so as to bond the surfaces together.

The two halves of the mould are preferably formed so as to have
externally projecting flanges extending along corresponding sections
of their perimeters the faces of which come into contact with the
two halves of the mould are brought together.  Most preferably these
flanges extend around the whole of the perimeter of the mould
except that portion which defines the opening.

The two halves of the mould are most preferably vacuum formed
but other techniques such as injection forming may be employed.
One or both halves of the mould may be formed so as to impress a
decorative design onto their surface which will be impressed onto
the surface of the air freshener thereby adding to the appeal of the
final product.

In a particularly preferred embodiment the flange on the half
of the mould extends further from the perimeter than the corresponding
flange on the opposite half.  When the two halves are brought together
this extension forms an overlapping portion which can be used to fix
the mould to a suitable support.  Conveniently this support is a piece
of cardborad or other such packaging material in which a hole or recess
is provided for the mould and at least the overlapping portion of the
flange extends over the edge of the hole or recess.  The mould is
then mounted on the support by fixing the flange to the support.
In a preferred embodiment the surface of the support which will come
into contact with the flange if treated with a suitable lacquer so
that when the flanges are joined to one another by heat treatment the
mould is also fixed to the support.

The support may be cut so that when the mould is placed in
position only the overlapping extended portion of the flange comes

BAD ORIGINAL

into contact with the support but preferably it will be cut so that
both of the flanges extending from the separate halves of the mould
contact the surface of the support.

Techniques of this nature are known as blister packaging in
the art but as far as the applicants are aware such techniques have
previously only been applied to preformed products and blister packages of this type have not previously been employed as moulds for
polymerisable materials.

The opening to the mould may be closed by sealing the sides of
the opening together or by providing a base member which is formed
from a material which is impermeable to the fragrance and inert to
the polymer. Preferably the mould is formed so as to have a relatively
narrow opening so as to minimise the surface of the polymer which is
in contact with air during the polymerisation. The opening will be
closed shortly after polymerisation is complete so as to minimise the
premature loss of the volatile ingredient. The base of the air
freshener is of a substantially rigid construction and is shaped so
as to fit snugly into the opening in the mould. The loss of fragrance
material by virtue of the solid air freshener being in contact with
the atmosphere is thereby minimised. The base is impermeable to the
fragrance material since if it is not we have found that fragrance
will be lost while the freshener is stored. Preferably the base
is formed from polyacrylic material.

The base may be joined to the air freshener composition by
any convenient means e.g. by a suitable adhesive. However, most
preferably the base is provided with one or more protruding members
on its inner surface and is placed in position in the opening of
the mould before the air freshener composition has become completely
solid. The protruding members thereby extend into the air freshener
composition and as it becomes more solid they are rigidly fixed in
position thereby holding the base firmly. These members need not
protrude to any great depth in order to fix the base. Preferably
they are constructed so that their cross-sectional area increases

0011924

the further they protrude from the base. Most preferably they are of mushroom shaped cross-section having a relatively broad cap mounted upon a corresponding narrow stem. Once the air freshener is set solid protruding members of this type can hold the base firmly in position.

The face of the base which is opposed to that which is in contact with the polymeric composition will normally be flat so that the finished product will stand upright on the base member when the latter is placed upon a flat surface. However, the base member may be fitted with a variety of attachment means which will enable the finished product to be mounted in a number of positions. Preferably such attachment means will be formed as an integral part of the structure of the base member so as to simplify the manufacture of the finished article. The aforesaid attachment means may take the form of a hook, a loop affixed to or a hole bored into the side and perhaps through the base which can engage a hook or protrusion, suction means e.g. suction cups or comprise an adhesive layer. Such attachment means may vary in their spatial relationship to the polymerised product so that the latter can be mounted upon a vertical surface as well as on a horizontal surface.

Where the base member is not used to close the mould the sealed product will normally be sold in conjunction with a suitable supporting member. Such a support will preferably contain a groove or recess into which the polymeric products can be inserted and retained although other means of support e.g. chips could be employed. Such supports will also be made from a material which is impermeable to the fragrance such as polyacrylic material.

Any polymeric packaging film which is capable of being formed into such a mould or blister pack and which fulfils the requirements described above can be used in this aspect of our invention. For example any of the specific polymers or laminates described herein are capable of being vacuum formed to provide such moulds or blister packs provided a film of suitable gauge e.g. 50 to 500 microns, more usually 100 to 300 microns is employed.

BAD ORIGINAL

0011924

Where polymerisation is to be carried out inside the package
the packaging material must be substantially impermeable to oxygen.
Films employed will have an air permeability of less than 100
preferably less than 10 and most preferably less than 2 $cm^3/m^2$ -
day-bar.

The complete package may conveniently be closed by "heat
sealing". Such techniques require that the inner face of the packaging
film be formed from a suitable polymeric material e.g. polyethylene
and where a laminate film is employed it is necessary that the inner
surface be formed of such a material if the package is to be closed
by heat sealing.

Alternatively, if the inner surface is not heat sealable
techniques such as ultrasonics and radio-frequency sealing can be
used to close the blister pack where the inner face of the packaging
film is formed from a material which is susceptible to these methods
of closure.

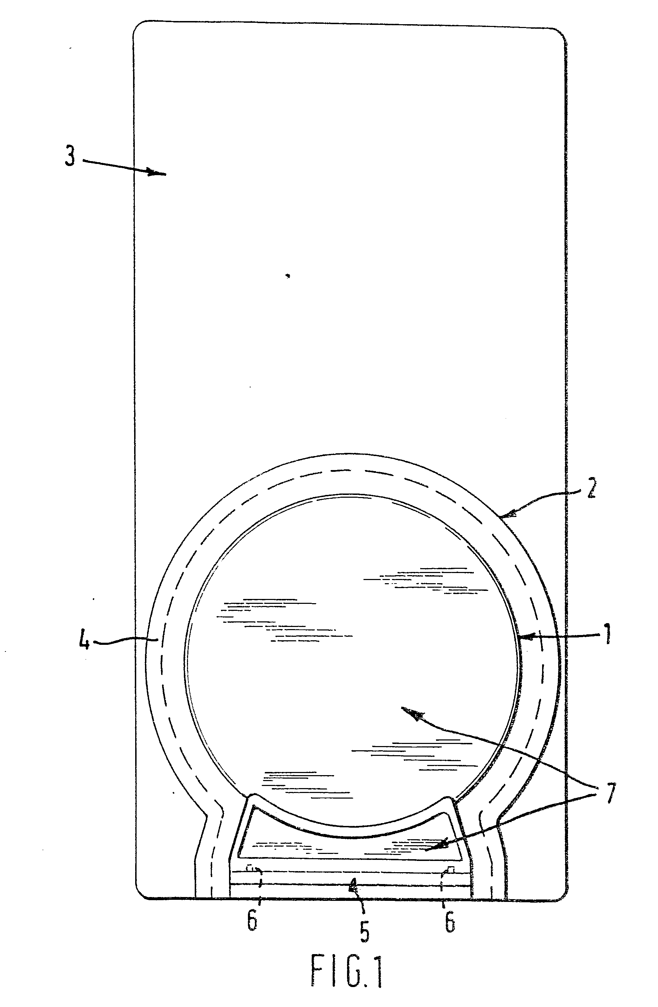

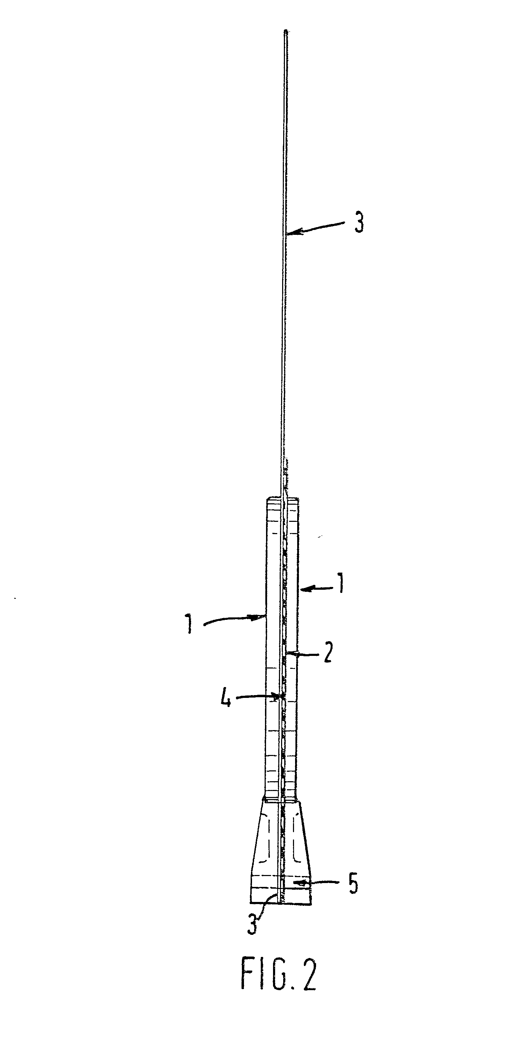

A finished product of the aspect of our invention concerned
with processes for the manufacture of polymeric fragrance products
is shown in the accompanying drawings. Figure 1 which shows the
product as a front elevation and Figure 2 showing a side elevation.
A hollow mould is formed from two separate halves of transparent
200 micron PVC film. The upper half has a flange 2 which extends
onto the surface of a carboard supporting member 3. The lower
half of the mould has a smaller flange 4 (shown as a dashed line)
lying wholly underneath the flange 2 but above the supporting
member 3.

The opening in the mould is closed by a rigid member 5
having two protrusions 6 and 6' which extend into the volume 7
which is occupied by the air freshener.

The improved exudation properties of a product of our invention is illustrated in the following Example.

EXAMPLE ONE

Two fragrances (A and B), manufactured by Bush Boake Allen Limited were used in this example. Mixtures of the following compositions were prepared. The quantities stated are by volume.

|  | Flower Shop | Honeysuckle |
|---|---|---|
| Hydroxypropyl methacrylate | 20.0 | 20.0 |
| Trimethylolpropane trimethacrylate | 2.5 | 2.5 |
| Fragrance A | 6.0 | - |
| Fragrance B | - | 6.0 |
| Polyoxyethylene (9) nonylphenol | 10.0 | 10.0 |
| 20% w/v aqueous ascorbic acid | 1.0 | 1.0 |
| Water | - | 1.0 |
| 70% hydrogen peroxide | 0.075 | 0.1 |
| 4% w/v copper sulphate in HCl | 0.025 | 0.025 |

These were tested in packages made from films based on polyvinyl chloride of the following three types:

a)    Uncoated rigid PVC, 200 micron thickness.

b)    PVC film similar to (a) coated on one side with polyethylene (30 micron thickness).

c)    PVC film similar to (a) coated on one side with polyethylene (30 micron thickness) and on the other with polyvinylidene chloride (40 g./sq. metre).

The packages were in the form of "blisters" sealed except for a filling aperture. They were filled through the latter with the liquid monomer mixtures which were allowed to polymerise, and the apertures were then sealed. The packages were hung in an oven at 45°C. After 3 days they were opened and the condition of the contents assessed by an experimental operative on the base of feel and appearance. The result is expressed using the following citeria:-

- SLIGHT   -   a noticeably greasy surface
   MODERATE -   greasy wet surface
   BAD     -   an obviously wet surface.

## FRAGRANCE A

a)    Uncoated PVC..................... Moderate exudation
b)    PVC/polyethylene ................ Slightly exudation
c)    PVC/polyethylene/PVC ............ No exudation

## FRAGRANCE B

a)    Uncoated PVC .................... Bad exudation
b)    PVC/polyethylene ................ Moderate exudation
c)    PVC/polyethylene/PVDC ........... No exudation

WHAT WE CLAIM IS:

1. A synthetic hydrophilic polymeric composition containing a volatile active ingredient which is capable of controlled release to the atmosphere and a package enclosing said polymeric composition to an extend sufficient substantially to prevent loss of active ingredient said package comprising a transparent film which is substantially impermeable to the active ingredient and which over at least part of the surface of the package constitutes the sole barrier between the polymeric composition and the outside atmosphere characterised in that the film has a water permeability of less than 5 $gm/m^2$ per day at $38^{o}C$ and 90% RH and which is sufficiently low to prevent exudation of the active ingredient on storage.

2. A composition according to claim 1, characterised in that the film has a water transmission of less than 3 $gm/m^2$ per day at $38^{o}C$ and 90% RH.

3. A composition according to claim 2 characterised in that the film has a water transmission of less than 2 $gm/m^2$ per day at $38^{o}C$ and 90% RH.

4.    A composition according to claim 2, characterised in that
      the film is a film selected from the group consisting of
      Barex/polythene and Barex/polypropylene duplex laminates,
      PVC/PVDC duples laminates PV/PVDC/polyethylene and PVC/
      PVDC/polypropylene triplex laminates and polyester/PVC/
      polyethylene triplex laminates.

5.    A synthetic polymeric composition containing a volatile
      active ingredient which is capable of controlled release
      into the atmosphere in a package formed from substantially
      air-impermeable film which has been formed by a process
      of simultaneously manufacturing and packing which process
      is characterised by the steps of (a) two pieces of
      substantially air-impermeable film being shaped to form
      opposite halves of a hollow mould having an opening at one
      end, said pieces being joined to form the mould, a mixture
      which is capable of forming the synthetic polymeric
      composition being introduced into the mould and the
      opening being filed with a member which is adopted to
      form a base for the composition and is inert to the
      ingredients thereof said member being joined to said
      polymeric composition during or after the polymerisation
      thereof.

6. A composition according to claim 5 formed by a process characterised in that the two halves of the mould have externally projecting flanges along corresponding sections of their perimeters which come into contact when the two halves are brought together.

7. A composition according to claim 6, formed by a process characterised in that the flange projecting from one half of the mould extends beyond that projecting from the opposite half.

8. A composition according to either of claims 6 or 7 which has been formed by a process characterised in that the flange is brought into contact with a suitable support so as to fix the product thereto.

9. A composition according to claim 8 characterised in that the flanges are formed from a material or coated with a material such that the two halves of the mould and the flanges thereof and the support can be jointed together by a single heat treatment step.

10. A composition according to claims 5 characterised in that the member adopted to form the base is formed from a polyacrylic material.

11. A composition according to claim 5 wherein the opening in the mould is closed by the base member prior to the polymerisation step characterised in that one or more protruding members extend from the inner face thereof to a point below the surface of the liquid prepolymer.

12. A composition according to claim 11, characterised in that the cross-sectional area of the protruding members increases with the distance from the base.

13. A composition according to claim 12, characterised in that said protruding members take the form of a mushroom shaped cap mounted upon a relatively narrow stem.

14. A composition according to claim 2, characterised in that synthetic polymer compositions are polymers of alpha-beta or Beta-gamma unsaturated carboxylic esters which possess hydrophilic functions.

15. A composition according to claim 14, characterised in that said esters possess a hydroxy function.

16. A composition according to claim 15, characterised in that the monomer is elected from the group consisting of hydroxyethyl acrylate, and methacrylate and the hydroxypropyl, hydroxybutyl or glceryl esters of acrylic or methacrylic acid.

17. A composition according to claim 15, characterised in that said monomer mixture contains a minor proportion of bi or poly functional monomers.

18. A composition according to claim 14, characterised in that the polymer comprises from 1 to 40% by weight of water.

19. A composition according to claim 18, characterised in that the polymer comprises from 2 to 10% by weight of water.

20. A composition according to claim 14, characterised in that the product comprises from 2.5 to 100% by weight of the polymer of the volatile active ingredient.

21.     A composition according to claim 20 characterised in that
        the product comprises from 5 to 50% by weight of the polymer
        of the volatile active material.

0011924

FIG.1

FIG.2

| Category | Citation of document with indication, where appropriate, of relevant passages | Relevant to claim |
|---|---|---|
| X | GB - A - 1 336 495 (CIBA-GEIGY) <br> * Page 7, lines 84-130; page 4, lines 79-119; page 33, claim 1 * | 1-4 |
| X | FR - A - 2 380 328 (BUSH BOAKE ALLEN) <br> * Page 18, claims * | 14-21 |
|  | FR - A - 1 580 871 (PECHINEY PROGIL) <br> * Page 3, abstract * | 1-4 |
|  | CH - A - 572 804 (DOW) <br> * Column 4, claim 1 * | 1-4 |

**DOCUMENTS CONSIDERED TO BE RELEVANT**

CLASSIFICATION OF THE APPLICATION (Int Cl )

A 61 L 9/12
B 65 D 81/24

TECHNICAL FIELDS SEARCHED (Int.Cl )

A 61 L 9/12
        9/04
B 65 D 81/24
A 01 N 25/18

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 11-03-1980 | PELTRE |

EPO Form 1503.1 06.78